Europäisches Patentamt

European Patent Office

Office européen des brevets

(19)

(11) Publication number: 0 278 994
A1

(12) EUROPEAN PATENT APPLICATION

(21) Application number: 87102168.9

(22) Date of filing: 16.02.87

(51) Int. Cl.⁴: A23G 3/30

(43) Date of publication of application:
24.08.88 Bulletin 88/34

(84) Designated Contracting States:
AT BE CH ES GR LI NL SE

(71) Applicant: ALFA GUM INVESTMENT INC.
Comosa Building Avenue
Panama City(PA)

(72) Inventor: Cappelari, Roberto
Via Cimabue 2
I-36061 Bassano del Grappa (Vicenza)(IT)

(74) Representative: Modiano, Guido et al
MODIANO, JOSIF, PISANTY & STAUB
Modiano & Associati Via Meravigli, 16
I-20123 Milan(IT)

(54) Chewing gum.

(57) This chewing gum may maintain constant the taste intensity over a long time or change its taste during the time by virtue of the fact that the gummy mass (2) is provided, inside, with a plurality of microcapsules (3) dispersed therein. Each microcapsule (3) contains active alimentary, confectionary or medical substances (8). Furthermore the microcapsules (3) are made of materials suitable for releasing the substances contained therein after preset times.

Fig.6

Fig.3

EP 0 278 994 A1

## CHEWING GUM

The present invention relates to a chewing gum. Currently, numerous products are known in the field which use flavors and, generally, substances suitable to give to the product particular tastes and fragrances.

In the particular case of chewing gums, the flavor-taste is contained in a simple acqueous solution which is immediately released right from the first mastications.

This is a disadvantage, since the released flavor or taste decreases at very low levels already after a few minutes of mastication, and the peculiar characteristics of these products thus is lost in a short time.

The aim of the present invention is to eliminate the disadvantages described above in known kinds, by providing a chewing gum which allows to maintain constant for preset times the taste intensity of active substances, such as alimentary, confectionary and/or medical, contained therein.

Within the scope of the above described aim, an important object is to provide a chewing gum which furthermore releases in a preset time sequence one or more of said active alimentary, confectionary and/or medical substances.

Another important object is to obtain a chewing gum which allows to program the release of said substances according to a preset time curve.

Not least object is to obtain a chewing gum which adds to the preceding characteristics that of being pleasant for mastication.

The aim and the objects described above are achieved by a chewing gum according to the appended claims.

The features and advantages of the invention will become apparent from the description of a particular, but not exclusive, embodiment, illustrated by way of non-limitative example in the accompanying drawing, where:

Fig. 1 shows the amount of available substances versus time in a conventional chewing gum;

Fig. 2 is a three-quarter view of a chewing gum;

Fig. 3 is a cross-section view along the plane III-III of Fig. 2;

Fig. 4 is a cross-section view of an ideal microcapsule; and

Figs. 5 to 7 show diagram similar to Fig 1 for the intentive chewing gum with different types of microcapsules.

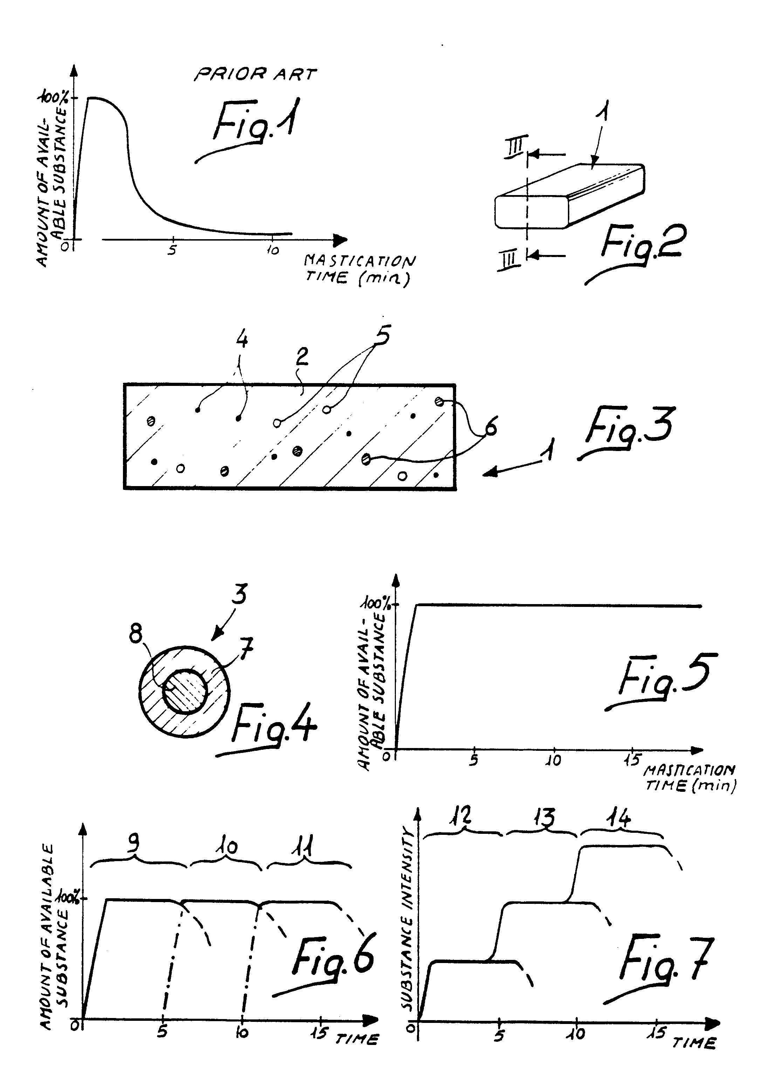

With reference to the above drawings, in Fig. 1 the usual time-based decay condition of the amount of substance available in chewing gums of the known kind is shown.

It can thus be observed that the flavor or the taste is immediately released right from the first mastications, its percentage dropping to extremely low values already a few minutes later.

On the contrary, the present invention regards a chewing gum 1 formed by a gummy mass 2 accommodating inside a plurality of microcapsules 3 which in the particular embodiment have been divided into three separate groups indicated at 4, 5 and 6.

Each microcapsule 3 presents extremely small dimensions, generally less than two-tenths of a millimeter, and are composed of a layer 7 of material suitable for maintaining the integrity of the same during mastication for preset time.

Internally to each microcapsule 3, a substance 8 is present, this substance being possibly of the alimentary, confectionary and/or medical type.

The materials composing the layer 7 can be, as an example, jelly, cellulose compounds, polyvinylpyrrolydone, starch, saccharose, lactose and others in various associations.

In Fig. 5 is illustrated the diagram relating to a particular embodiment of the invention: it relates to a chewing gum including groups of microcapsules containing, as an example, the same substance 8, this substance being released, within the different groups, in periods which are subsequent and such as to allow the availability of the desired substance for the time required, which, in theory, can be indefinite.

In Fig. 6 is illustrated the diagram of a second embodiment of the invention, wherein the chewing gum includes the three different groups 4, 5 and 6 of microcapsules containing each different substances 8 and having each a different releasing time by virtue of the different disintegration times of the layers 7.

During mastication, therefore, a first substance will be available in a first period 9, a second substance different in organoleptic or chemical characteristics will be available in the second period 10, and analogously for a third period 11.

In Fig. 7 is illustrated the curve relative to a further embodiment of the invention, wherein the chewing gum includes the different groups of microcapsules having different releasing times; however here the microcapsules 3 contain the same kind of substance 8, but in increasing quantities, the layer 7 allowing releasing of the substance 8 according to preset times.

Thus, for example, in a first period 12 a given amount of substance is available for a preset time, in a second period 13 the same substance is available with a larger amount, for a further preset time, and finally in a subsequent period 14 with a further increased substance quantity is available for a further preset period.

Therefore, as explained, the chewing gum according to the invention allows predetermination of the substance available during mastication both regarding its quantity, its taste and its duration in time. In particular, it is pointed out that the longer periods, in which a substance contained originally in the same group of microcapsules is available, are obtained thanks to the presence of the layer 7 by selecting suitable materials therefor, and by virtue of the statistic distribution of the disintegration times of the layers 7 of a same microcapsules group.

Naturally, this substance may represent either a taste and flavor modifier, of a pharmaceutical substance, for example for oral hygiene and for giving fluoride to the dental apparatus.

Furthermore the chewing gum according to the invention allows obtainment, as an example, of a taste which is constant even for prolonged mastication periods or of a taste the intensity of which increases in time, or still of different tastes for subsequent mastication periods.

The dimensions of the microcapsules are such as not to be felt by the tongue during mastication, avoiding any unpleasant feeling.

Naturally the invention thus conceived is susceptible of numerous modifications and variations, without departing from the scope of the same inventive concept.

Thus, the number of microcapsules present inside the chewing gum, and the number of microcapsules belonging to the same group, may be any.

Furthermore, the kind of substances used and their amounts both within each microcapsule and within each group, may be any according to the requirements.

## Claims

1. Chewing gum comprising a gummy mass (2), characterized in that inside said gummy mass (2) a plurality of microcapsules (3) is dispersed, said microcapsules containing active alimentary, confectionary and/or medical substances (8), said microcapsules (3) releasing said substances (8) at preset times.

2. Chewing gum according to claim 1, characterized in that said microcapsules (3) present an outer layer (7) of mastication resistant material, thereby said layers remaining intact and avoiding said substances from spreading during the mastication for a preset time.

3. Chewing gum according to claim 1, characterized in that said active substances (8) are of the alimentary, the confectionary and/or of the pharmaceutical type, said substances (8) being present in preset amounts and/or with different organoleptic characteristics.

4. Chewing gum according to one or more of the preceding claims, characterized in that it comprises, more groups of microcapsules, having different mastication resistance features and/or different substances in preset amounts.

5. Chewing gum according to one or more of the preceding claims, characterized in that said outer layers are made of a material selected from the group comprising jelly, cellulose compounds, polyvinylpyrrolydone, starch, saccharose, lactose, and a combination thereof.

6. Chewing gum according to one or more of the preceding claims, characterized in that said microcapsules have dimensions smaller than two-tenths of a millimeter.

0 278 994

PRIOR ART

Fig.1

AMOUNT OF AVAIL-ABLE SUBSTANCE %

100%

5    10    MASTICATION TIME (min)

Fig.2

Fig.3

Fig.4

Fig.5

AMOUNT OF AVAIL-ABLE SUBSTANCE

100%

5    10    15    MASTICATION TIME (min)

Fig.6

AMOUNT OF AVAILABLE SUBSTANCE

100%

9    10    11

5    10    15    TIME

Fig.7

SUBSTANCE INTENSITY

12    13    14

5    10    15    TIME

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP-A-0 123 524 (WARNER-LAMBERT) * Claims 1,9,10; page 9, line 4 - page 10, line 3; page 11, lines 24-27 * | 1-6 | A 23 G   3/30 |
| X | US-A-4 386 106 (C.G. MERRITT et al.) * Claims 1,16,17,19,22; column 12, lines 27-31 * | 1-6 | |
| E,L | EP-A-0 217 109 (R. CAPPELLARI) * Abstract, paragraph 3; columns 1-4, figures 1-7 * | 1-6 | |
| X | US-A-3 826 847 (K. OGAWA et al.) * Example 6; column 1, line 53 - column 2, line 8; column 3, lines 1-6 * | 1-6 | TECHNICAL FIELDS SEARCHED (Int. Cl.4) |
| A | FR-A-2 413 039 (LIFE SAVERS) | | A 23 G |
| A | US-A-3 205 075 (WARNER-LAMBERT) | | |
| A | US-A-3 795 744 (K. OGAWA et al.) | | |
| | --- -/- | | |

The present search report has been drawn up for all claims

| Place of search | Date of completion of the search | Examiner |
|---|---|---|
| THE HAGUE | 12-11-1987 | GUYON R.H. |

| DOCUMENTS CONSIDERED TO BE RELEVANT | | | Page 2 |
|---|---|---|---|
| Category | Citation of document with indication, where appropriate, of relevant passages | Relevant to claim | CLASSIFICATION OF THE APPLICATION (Int. Cl.4) |
| A | FR-A-1 228 510 (GENERAL FOODS CORP.) | | |
| A | US-A-4 238 475 (F. WITZEL et al.) | | |
| A | US-A-4 259 355 (DON MARMO et al.) | | |
| A | US-A-4 384 004 (T. CEA) * Example 1, table II,I * | 1-3,5 | |

TECHNICAL FIELDS SEARCHED (Int. Cl.4)

The present search report has been drawn up for all claims

| Place of search | Date of completion of the search | Examiner |
|---|---|---|
| THE HAGUE | 12-11-1987 | GUYON R.H. |